# EUROPEAN PATENT SPECIFICATION

(11) **EP 0 719 263 B1**
(45) Date of publication and mention of the grant of the patent: **15.07.1998**
(21) Application number: 94926240.6
(22) Date of filing: 05.09.1994
(51) Int. Cl.: C07D 333/42, C07D 307/73, C07D 271/12, C07C 217/84, A61K 31/38, A61K 31/345, A61K 31/41

(54) **HETEROCYCLIC CARBOXAMIDE DERIVATIVES WITH ANTIARRHYTHMIC ACTIVITY**
HETEROCYCLISCHE CARBOXAMIDDERIVATE MIT ANTIARRYTHMISCHER WIRKUNG
DERIVES HETEROCYCLIQUES DE CARBOXAMIDE AYANT UNE ACTIVITE ANTIARYTHMIQUE

(30) Priority: 13.09.1993 FR 9310849
(43) Date of publication of application: 03.07.1996
(73) Proprietor: SMITHKLINE BEECHAM LABORATOIRES PHARMACEUTIQUES, 92731 Nanterre Cédex (FR)
(72) Inventor: NADLER, Guy, Marguerite, Marie, Gérard SmithKline, F-35762 Saint-Grégoire (FR)
(74) Representative: Rutter, Keith, Dr.
(86) International application number: EP9402960
(87) International publication number: WO9507903

(56) References cited:
- EP-A- 0 233 762
- EP-A- 0 431 944
- FR-A- 2 636 631
- FR-A- 2 691 064

## Description

The invention relates to certain novel compounds, to pharmaceutical compositions containing such compounds, to a process for the preparation of such compounds and to the use of such compounds as active therapeutic agents, particularly in the treatment of atrial or ventricular cardiac arrhythmias.

Anti-arrhythmic agents are classified according to their electrophysiological effects on the cardiac cell (Vaugham-Williams, 1970, 1989) : class I agents block the fast sodium current, class II agents are beta-adrenergic blockers, class III agents block potassium currents, class IV agents block the calcium current, and class V agents are specific sinus node inhibitors.

A majority of ventricular and atrial arrhythmias are related to reentrant circuit. The prolongation of myocardial refractoriness within or surrounding such a reentrant circuit is a potential mechanism for the management of cardiac arrhythmias.

Because class III antiarrhythmic agents block cardiac potassium currents, they prolong the repolarisation process and increase refractoriness. Consequently class III agents represent the most specific class to treat reentrant arrhythmias.

However, due to their mechanism of action, i.e. a concentration dependent increase in the cardiac action potential duration, higher doses of class III antiarrhythmic agents may trigger arrhythmias. Such arrhythmias, called Torsade de Pointe generally represents the main adverse effect for all pure class III compound currently in development.

European Patent Application, Publication Number 0233762 discloses a class of compounds of formula A 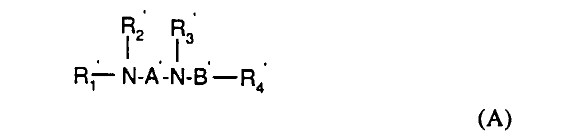 wherein
R₁' and R₄' are independently phenyl optionally substituted by one, two or three of halogen, trifluoromethyl, C₁₋₄ alkoxy, C₁₋₄ alkyl, cyano, hydroxy, nitro, NR₅'R₆' or O₂SNR₅'R₆' wherein R₅' and R₆' are independently hydrogen or C₁₋₆ alkyl or together are C₃₋₆ polymethylene, or disubstituted at adjacent carbon atoms by C₁₋₂ alkylenedioxy and optionally further substituted by one of the above groups;
R₂' is selected from (CH₂)_{z} CN where z is zero or an integer from 1 to 4, C₁₋₁₂ alkyl,
C₃₋₇ cycloalkyl, C₃₋₇ cycloalkyl C₁₋₄ alkyl, phenyl C₁₋₄ alkyl, pyridyl, pyridyl C₁₋ ₄ alkyl, COR₇', COCH₂COR₇', SO₂R₇', CO₂R₇', CONHR₇' and CSNHR₇', where R₇' is selected from C₃₋₁₂ alkyl, C₃₋₇ cycloalkyl, C₃₋₇ cycloalkyl C₁₋₄ alkyl, phenyl and phenyl C₁₋₄ alkyl, any alkyl moiety in R₇' optionally substituted by hydroxy or C₁₋₄ alkanoyloxy, any pyridyl or phenyl moiety in R₂' optionally substituted as defined for R₁' and R₄' and any cycloalkyl moiety in R₂' optionally substituted by one or two C₁₋₄ alkyl groups;
R₃' is hydrogen or C₁₋₄ alkyl;
A' represents C₂₋₅ alkylene; and
B' represents C₁₋₄ alkylene.

These compounds are stated to possess cardiovascular activity, in particular calcium antagonistic activity which indicates that they are of potential use in the treatment of angina pectoris. In WO 93/23024 certain of the compounds of formula (A) are disclosed as having both class III and class IV antiarrhythmic activity.

It has now been discovered that certain heterocyclic amides, structurally distinct from the compounds of formula (A), induce a self-limiting increase of the cardiac action potential duration, related to a dual blockade of cardiac potassium and calcium channels. Consequently, they have an improved pharmacological profile over pure class III anti-arrhythmic agents, in particular they are indicated to have low proarrhytmic potential and to restore the contractile function of the ischaemic myocardium.

Accordingly, the invention relates to a compound of formula (I): 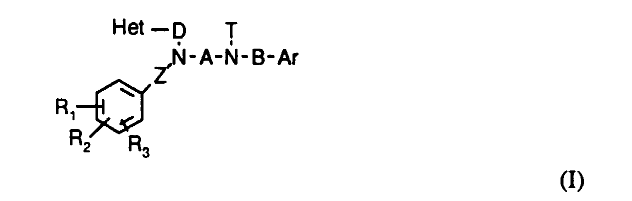 or a salt thereof, or a solvate thereof, wherein
A and B each independently represents a C₁₋₄ n-alkylene group wherein each carbon is optionally substituted by a C₁₋₆ alkyl group;
D represents CO, SO₂, NH-CO or CH₂CO;
T represents hydrogen, alkyl, alkene or cycloalkyl; and
Het represents substituted or unsubstituted: furanyl, pyranyl, thienyl, thiazolyl, imidazolyl, triazolyl or the benzo fused equivalents of furanyl, pyranyl, thienyl, thiazolyl, imidazolyl or triazolyl, indolyl, oxoindolyl, indenyl, isoindenyl, indazolyl, indolizinyl, or benzofurazanyl;
optional substituents for the moiety Het include 1 to 5 substituents selected from the list consisting of nitro, halogen, alkylsulfonamido, alkylamido, 1H-imidazolyl, alkyl and haloalkyl;
R₁, R₂ and R₃ each independently represent H, alkyl, OH or alkoxy or, if attached to adjacent carbon atoms, any two of R₁, R₂ or R₃ together with the carbon atoms to which they are attached may form a fused heterocyclic ring of five to six atoms wherein one, two or three of the said atoms are oxygen or nitrogen;
Z represents a bond, CH₂, (CH₂)₂ or X-CH₂-CH₂ wherein X represents O or S; and Ar represents substituted or unsubstituted aryl, wherein the optional substituents are the above defined R₁, R₂ and R₃.

Suitably, A represents (CH₂)₃.

Suitably, B represents CH₂CH₂.

Suitably, D represents CO, SO₂, NH-CO or CH₂CO.

Suitably, T represents CH₃.

Suitably, one or two of R₁, R₂ and R₃ represents alkoxy, for example methoxy, the remaining member(s) being H.

Suitably, Het includes furanyl and thienyl.

Suitably Het is sustituted by 1,2 or 3 substituents, favourably 1.

An example of a substitutent for Het is a nitro group.

Examples of Het are 4-nitro-2-thiophenyl, 5-nitro-2-furanyl and 5-benzofurazanyl.

As used herein, unless indicated to the contrary, the term "alkyl" includes straight or branched chain alkyl groups having from 1 to 12, favourably 1 to 6, carbon atoms and shall include such alkyl groups when forming part of other groups such as alkoxy or arylalkyl groups.

As used herein, unless indicated to the contrary, the term "alkenyl" includes straight or branched chain alkylene groups having from 2 to 12, favourably 2 to 6, carbon atoms and one or more double bonds.

As used herein, unless indicated to the contrary, the term "aryl" includes phenyl and naphthyl, preferably phenyl.

As used herein, unless indicated to the contrary, the term "cycloalkyl" includes C₃₋₈ preferably C₄₋₆ cycloalkyl groups.

As used herein "halogen" includes fluorine, chlorine or bromine.

As used herein, the term "cardiac arrhythmia" relates to any variation from the normal rhythm of heart beat, including, without limitation, sinus arrhythmia, premature heartbeat, heartblock, fibrillation, flutter, tachycardia, paroxysmal tachycardia and premature ventricular contractions.

The compounds of formula (I) may possess chiral carbon atoms and therefore may exist in more than one stereoisomeric form. The invention extends to any of the stereoisometric forms, including enantiomers of the compounds of formula (I) and to mixtures thereof, including racemates. The different stereoisomeric forms may be separated or resolved one from the other by conventional methods or any given isomer may be obtained by conventional stereospecific or asymmetric syntheses.

The pharmaceutically acceptable salts of the compounds of formula (I) include acid addition salts with pharmaceutically acceptable mineral acids such as hydrochloric, hydrobromic, boric, phosphoric, sulphuric and pharmaceutically acceptable organic acids such as acetic, tartaric, maleic, citric, succinic, benzoic, ascorbic, methanesulphonic, a-keto-glutaric, a-glycerophosphoric, and glucose-1-phosphoric acids. Preferably the acid addition salt is a hydrochloride.

Pharmaceutically acceptable salts also include quaternary salts. Examples of quaternary salts include such compounds quaternised by compounds such as R^{z}-T¹ wherein R^{z} is C₁₋₆ alkyl, phenyl-C₁₋₆ alkyl or C₅₋₇ cycloalkyl, and T¹ is a radical corresponding to an anion of an acid. Suitable examples of R^{z} include methyl, ethyl and *n*- and *iso-* propyl; and benzyl and phenethyl. Suitably T¹ includes halide such as chloride, bromide and iodide.

Pharmaceutically acceptable salts also include pharmaceutically acceptable N-oxides, and the invention extends to these.

The compounds of the formula (I) and their salts may also form solvates, especially pharmaceutically acceptable solvates, such as hydrates, and the invention extends to these, and especially to the pharmaceutically acceptable solvates.

The salts of the compounds of the formula (I) which are not pharmaceutically acceptable may be useful as intermediates in the preparation of pharmceutically acceptable salts of compounds of formula (I) or the compounds of the formula (I) themselves, and as such form an aspect of the present invention.

A compound of formula (I) or a salt thereof, or a solvate thereof, may be prepared by reacting a compound of formula (II): 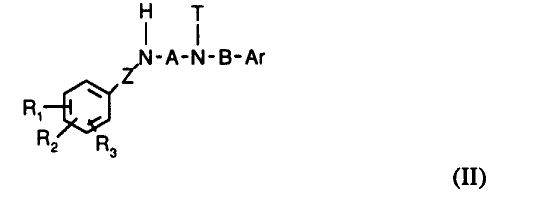 wherein A, B, T, Z, R₁, R₂, R₃ and Ar are as defined in relation to formula (I) with a compound of formula (III);

Het-X (III)

wherein Het is as defined in relation to formula (I) and:
(a) for compounds of formula (I) wherein D is CO or SO₂, X represents CO.L₁ or SO₂.L₁ respectively wherein L₁ is a leaving group or atom such as a halogen atom; or
(b) for compounds of formula (I) wherein D is NH.CO, X is N=C=O; and thereafter, if required, carrying out one or more of the following optional steps:
   (i) converting a compound of formula (I) into a further compound of formula (I);
   (ii) preparing a salt of the compound of formula (I) and/or a solvate thereof.

The reaction conditions for the reaction between compounds of formulae (II) and (III) are conventional conditions appropriate to the nature of the reagent used, generally however the reaction may be carried out in an inert solvent, such as methylene chloride, at any suitable temperature providing a convenient rate of formation of the desired product, generally at an ambient to elevated temperature, conveniently at the reflux temperature of the solvent and preferably in the presence of a base such as triethylamine.

Compounds of formula (II) are known compounds or they may be obtained by procedures analogous to those used to prepare known compounds, for example as described in EP 0 233 762.

The compounds of formulae (III) are known commercially available compounds or they may be obtained by procedures analogous to those used to prepare known compounds, for example compounds of formula (III) wherein L¹ represents CO.X may be prepared according to methods described in Organic Synthesis, Coll. Vol. I page 394 or Organic Synthesis, Coll. Vol. III, page 29, compounds of formula (III) wherein L₁ represents SO₂.X may be prepared according to methods described in Organic Synthesis, Coll. Vol. VIII, page 104 and for compounds of formula (III) wherein L₁ represents N=C=O according to methods described in Organic Synthesis, Coll. Vol. III, page 846.

It will be appreciated that in any of the abovementioned reactions any reactive group in the substrate molecule may be protected, according to conventional chemical practice.

Suitable protecting groups in any of the abovementioned reactions are those used conventionally in the art. The methods of formation and removal of such protecting groups are those conventional methods appropriate to the molecule being protected.

As mentioned above the compounds of the invention are indicated as having useful therapeutic properties: The present invention accordingly provides a compound of formula (I), or a pharmaceutically acceptable salt thereof and/or a pharmaceutically acceptable solvate thereof, for use as an active therapeutic substance.

More particularly, the present invention provides a compound of formula (I), or a pharmaceutically acceptable salt thereof and/or a pharmaceutically acceptable solvate thereof, for use in the treatment of and/or prophylaxis of arrhythmia, especially cardiac arrhythmia such as ventricular arrhythmia, and also ischeamic rhythm disorders.

A compound of formula (I), or a pharmaceutically acceptable salt thereof and/or a pharmaceutically acceptable solvate thereof, may be administered per se or, preferably, as a pharmaceutical composition also comprising a pharmaceutically acceptable carrier.

Accordingly, the present invention also provides a pharmaceutical composition comprising a compound of the general formula (I), or a pharmaceutically acceptable salt thereof, or a pharmaceutically acceptable solvate thereof, and a pharmaceutically acceptable carrier thereof.

A compound of formula (I) or a pharmaceutically acceptable salt thereof and/or a pharmaceutically acceptable solvate thereof is normally administered in unit dosage form.

An amount effective to treat the disorder hereinbefore described depends upon such factors as the efficacy of a compound of formula (I) , the particular nature of the pharmaceutically acceptable salt or pharmaceutically acceptable solvate chosen, the nature and severity of the disorders being treated and the weight of the mammal. However, a unit dose will normally contain 1 to 50 mg for example 2 to 15 mg, of the compound of the invention.

Unit doses will normally be administered once or more than once a day, for example 2, 3, 4, 5 or 6 times a day, more usually 2 to 4 times a day, such that the total daily dose is normally in the range, for a 70 kg adult of 1 to 300 mg, more usually 4 to 100 mg, for example 10 to 60mg, that is in the range of approximately 0.02 to 5 mg/kg/day, more usually 0.1 to 2 mg/kg/day, for example 0.1 to 0.5 mg/kg/day.

In such treatment, the compound may be administered by any suitable route, e.g. by the oral, parenteral or topical routes. For such use, the compound will normally be employed in the form of a pharmaceutical composition in association with a human or veterinary pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

For topical administration, the composition may be in the form of a transdermal ointment or patch for systemic delivery of the compound and may be prepared in a conventional manner, for example, as described in the standard textbooks such as 'Dermatological Formulations' - B.W. Barry (Drugs and the Pharmaceutical Sciences - Dekker) or Harrys Cosmeticology (Leonard Hill Books).

In addition such compositions may contain further active agents such as anti-hypertensive agents and diuretics.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of arrhythmia, especially cardiac arrhythmia such as ventricular arrhythmia, and also ischemic rhythm disorders in a human or non-human mammal which comprises administering an effective, non-toxic, amount of a compound of the general formula (I), or a pharmaceutically acceptable salt thereof and/or a pharmaceutically acceptable solvate thereof to a human or non-human mammal in need thereof.

Conveniently, the active ingredient may be administered as a pharmaceutical composition hereinbefore defined, and this forms a particular aspect of the present invention.

In the treatment and/or prophylaxis of arrhythmia and/or ischeamic arrhythmia disorders the compound of the general formula (I), or a pharmaceutically acceptable salt thereof and/or a pharmaceutically acceptable solvate thereof, may be taken in doses, such as those described above.

Similar dosage regimens are suitable for the treatment and/or prophylaxis of non-human mammals.

In a further aspect the present invention provides the use of a compound of formula (I), or a pharmaceutically acceptable salt thereof and/or a pharmaceutically acceptable solvate thereof, for the manufacture of a medicament for the treatment of arrhythmia, especially cardiac arrhythmia such as ventricular arrhythmia, and also ischemic rhythm disorders.

The following Examples illustrate the invention but do not limit it in any way.

### Example 1

### N-(3,4-Dimethoxyphenyl)-N-[3-[[2-(3,4-dimethoxyphenyl]ethyl]methylamino]propyl]-4-nitro-2-thiophenecarboxamide, hydrochloride.

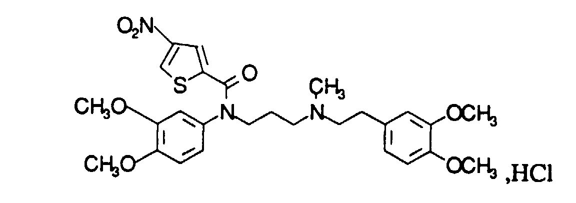

To an ice cooled stirred solution of 2.8 g (7.5 mmol) of N,-(3,4-dimethoxyphenyl)-N'-[3-[[2-(3,4-dimethoxyphenyl]ethyl]-N'-methyl-1,3-propanediamine and 0.75 g (7.5 mmol) of triethylamine in 40 ml of chloroform, 1.43 g (7.5 mmol) of 4-nitro-2-thiophenecarboxylic acid chloride were added dropwise over a period of 15 min..The mixture was left to come to room temperature and stirred for additional 4 hours. Then 100 ml of methylene chloride were added and the organic phase was washed three times with 100 ml water, dried over MgSO₄ and concentrated in vacuo. Purification by flash chromatography on silica gel using 98:2 ethyl acetate : methanol followed by concentration and trituration in ether yielded of a yellow crystalline product.The product was then dissolved in 20ml ethyl acetate, 0.25ml of 5.5N anhydrous HCl in ether was added.The mixture was concentrated in vacuo to afford 0.7g (23%) of a yellow solid.
mp.: 100°C
¹H NMR(DMSO-d6) d = 1.97 (m,2H,CH₂); 2.79 (s,3H,NCH₃); 2.93 (m,2H,CH₂); 3.24 (m,4H,CH₂x2); 3.72-3.85 (m,14H,OCH₃x4 + CH₂NCO); 6.76-7.15 (m,7H,Ar); 8.81 (d,1H,Ar); 10.30(s,1H,exch.D₂O,NH⁺)ppm.

### Example 2

### N-(3,4-Dimethoxyphenyl)-N-[3-[[2-(3,4-dimethoxyphenyl)ethyl]methylamino]propyl]-5-nitro-2-furancarboxamide, hydrochloride

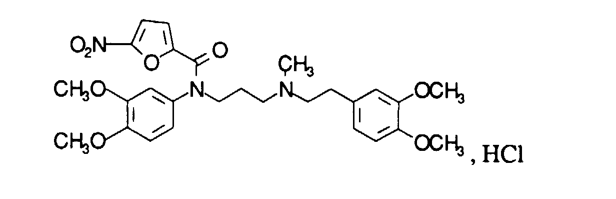

Starting from 800 mg (2.0 mmol) of N-(3,4-dimethoxyphenyl)-N'-[2-(3,4-dimethoxyphenyl) ethyl] -N'-methyl-1,3-propanediamine and 434 mg (2.5 mmol) of 5-nitro-2-furancarbonyl chloride and following the method described in example 1, yielded 560 mg (50%) of the title compound as a yellow solid.
The compound was purified as free base by two successive chromatographies on silica gel using methylene chloride:methanol first 98:2 then 99:1.
m.p = 98-111°C
¹H NMR(DMSO-***d6***) δ = 1.82-2.04 (m,2H,CH₂); 2.80 (d,3HJ=4.0Hz,CH₃NH); 2.85-3.00 (m,2H,CH₂); 3.08-3.43 (m,4H,CH₂**x2**); 3.73 (1s,6H,CH₃O**x2**); 3.75 and 3.79 (2s,6H,CH₃O**x2**); 3.75-3.93 (m,2H,CH₂); 5.82 (d,1H,J=3.6Hz,Ar); 6.78-7.13 (m,6H,Ar); 7.51 (d,1H,J=3.6Hz,Ar); 10.19 (s,1H,exchD₂O,NH⁺) ppm.

### Example 3

### N-(3,4-Dimethoxyphenyl)-N-[3-[[2-(3,4-dimethoxyphenyl)ethyl]methylamino]propyl]-5-benzofurazancarboxamide hydrochloride hydrate.

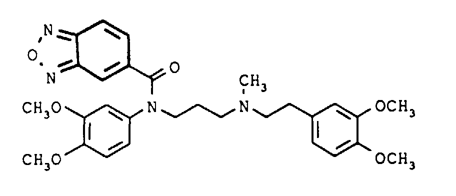

Starting from 680 mg (1.75 mmol) of N-3,4-dimethoxyphenyl)-N'-[2-(3,4-dimethoxyphenyl)ethyl]-N'-methyl-1,3-propanediamine and 306 mg (1.67 mmol) of 5-benzofurazancarbonyl chloride in 20 ml of methylene chloride and following the procedure described in example 1, yielded 414 mg (43%) of the title compound as a yellow solid.
The compound was purified as free base by flash chromatography on silica gel using methylene chloride: methanol 95:5
m.p. =90°C
¹H NMR(DMSO-d6) δ = 1.99(m, 2H, CH₂); 2.81(s, 3H, NCH₃); 2.95(m, 2H, CH₂); 3.27(m, 4H, CH₂x2); 3.64, 3.66, 3.73 and 3.75(4s, 12H, OCH₃x4); 3.94(m, 2H, CH₂NCO); 6.75-6.93(m, 5H, Ar); 7.06(s, 1H, Ar); 7.54(d, 1H, Ar); 7.92-8.02(m, 2H, Ar); 10.36(broad band, 1H, exch. D₂O, NH⁺)ppm.

### Pharmacological data

### Methodology

Guinea pigs (300-350 g) were anesthetized by intravenous injection of sodium pentobarbital (60 mg/kg). After thoracotomy the heart was rapidly excised and placed in oxygenated Tyrode solution. Papillary muscles were removed from the right ventricle. Preparations were then fixed to the silastic base of a 5 ml organ bath and superfused with oxygenated Tyrode solution maintained at 37 ± 1°C.
The modified Tyrode solution (pH 7.35) contained the following (mM) : NaCl 125, KCl 4.0, MgCl₂ 0.5, CaCl₂ 1.8, NaHCO₃ 24, NaH₂PO₄ 0.9 and glucose 5.5. The solution was equilibrated with a gas mixture of 95% O₂ - 5% CO₂.
After a stabilisation period (at least 1h), transmembrane action potentials were recorded with conventional microelectrodes (10 MOhm) connected to a high input impedance amplifier (BIOLOGIC VF 180). External stimuli were delivered to the preparation with bipolar platinum electrodes placed at one end of the muscle. The pulse duration was 1 ms and the amplitude was twice threshold. The basic cycle length was 1000 ms (PULSAR 6i stimulator). The signals were monitored on a storage oscilloscope (GOULD 1602) and simultaneously recorded on a digital tape recorder (BIOLOGIC DTR 1200) for further analysis.
Measurements were made on resting membrane potential (RMP), action potential amplitude (APA) and action potential durations at 30, 50, and 90% repolarization (APD30, APD50 and APD90 respectively). Recordings were made after 30 min of equilibration for each concentration. Only recordings in which the same impalement was maintained throughout the entire experiment were used for analysis.

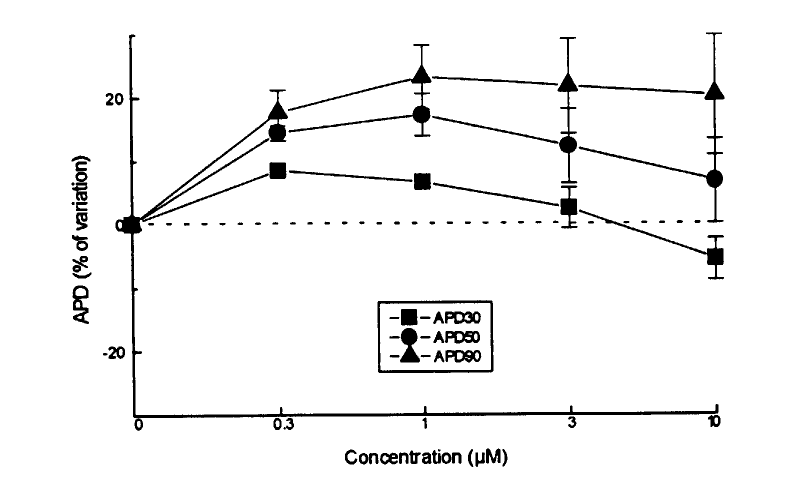

Effect of compound of example 1 on action potential duration (APD) recorded in guinea-pig papillary muscle. Action potential duration was mesured at 30% (APD30), 50%(APD50) and 90% (APD90) of repolarization.

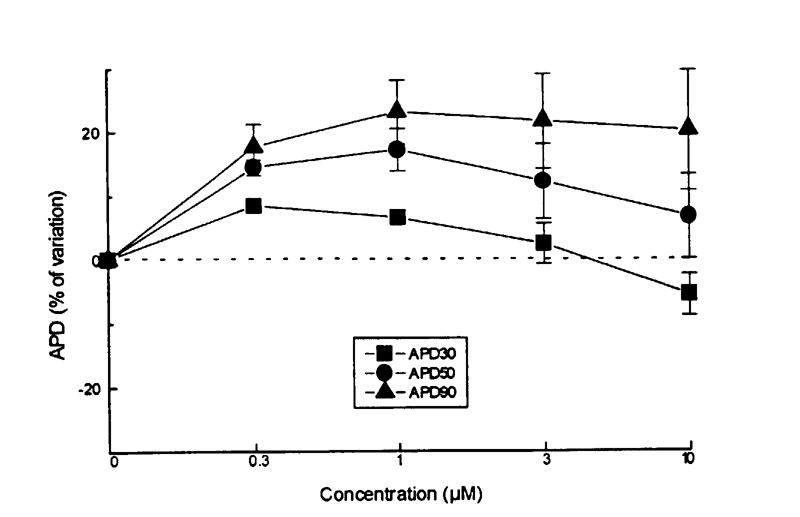

Effect of compound of example 2 on action potential duration (APD) recorded in guinea-pig papillary muscle. Action potential duration was mesured at 30% (APD30), 50%(APD50) and 90% (APD90) of repolarization.

## Claims

1. A compound of formula (I): 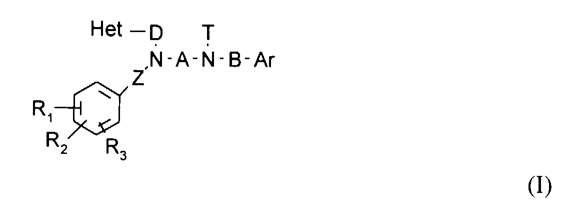 or a salt thereof, or a solvate thereof, wherein
A and B each independently represents a C₁₋₄ n-alkylene group wherein each carbon is optionally substituted by a C₁₋₆ alkyl group;
D represents CO, SO₂, NH-CO or CH₂CO;
T represents hydrogen, alkyl, alkene or cycloalkyl; and
Het represents substituted or unsubstituted: furanyl, pyranyl, thienyl, thiazolyl, imidazolyl, triazolyl or the benzo fused equivalents of furanyl, pyranyl, thienyl, thiazolyl, imidazolyl or triazolyl, indolyl, oxoindolyl, indenyl, isoindenyl, indazolyl, indolizinyl, or benzofurazanyl;
optional substituents for the moiety Het include 1 to 5 substituents selected from the list consisting of nitro, halogen, alkylsulfonamido, alkylamido, 1H-imidazolyl, alkyl and haloalkyl;
R₁, R₂ and R₃ each independently represent H, alkyl, OH or alkoxy or, if attached to adjacent carbon atoms, any two of R₁, R₂ or R₃ together with the carbon atoms to which they are attached may form a fused heterocyclic ring of five to six atoms wherein one, two or three of the said atoms are oxygen or nitrogen;
Z represents a bond, CH₂, (CH₂)₂ or X-CH₂-CH₂ wherein X represents O or S; and Ar represents substituted or unsubstituted aryl, wherein the optional substituents are the above defined R₁, R₂ and R₃.

2. A compound according to claim 1, wherein A represents (CH₂)₃, B represents CH₂CH₂ and T represents CH₃.

3. A compound according to claim 1 or claim 2, wherein D represents CO, SO₂ or NH-CO or CH₂CO.

4. A compound according to any one of claims 1 to 3 wherein D represents CO.

5. A compound according to any one of claims 1 to 4, wherein one or two of R₁, R₂ and R₃ represents alkoxy and the remaining member(s) each independently represent H.

6. A compound according to any one of claims 1 to 5, wherein Het represents furanyl or thienyl.

7. A compound according to any one of claims 1 to 6, wherein Het represents 4-nitro-2-thiophenyl, 5-nitro-2-furanyl or 5-benzofurazanyl.

8. A compound according to claim 1, being:
N-(3,4-dimethoxyphenyl)-N-[3-[[2-(3,4-dimethoxyphenyl]ethyl] methylamino]propyl]-4-nitro-2-thiophenecarboxamide;
N-(3,4-dimethoxyphenyl)-N-[3 -[[2-(3,4-dimethoxyphenyl)ethyl]methyl-amino]propyl]-5-nitro-2-furancarboxamide; and
N-(3,4-dimethoxyphenyl)-N-[3-[[2-(3,4-dimethoxyphenyl)ethyl]methylamino]propyl]-5-benzofurazancarboxamide hydrochloride hydrate; or a salt thereof. or a solvate thereof.

9. A process for the preparation of a compound of formula (I), which process comprises reacting a compound of formula (II): 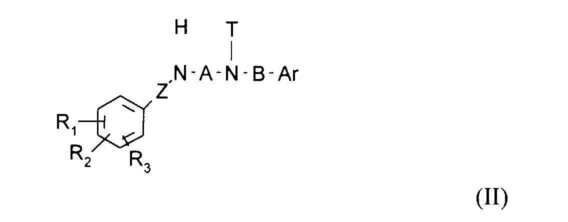 wherein A, B, T, Z, R₁, R₂, R₃ and Ar are as defined in relation to formula (I) with a compound of formula (III);
Het-X (III)
wherein Het is as defined in relation to formula (I) and:
(a) for compounds of formula (I) wherein D is CO or SO₂, X represents CO.L₁ or SO₂.L₁ respectively wherein L₁ is a leaving group or atom such as a halogen atom; or
(b) for compounds of formula (I) wherein D is NH.CO, X is N=C=O; and thereafter, if required, carrying out one or more of the following optional steps:
(i) converting a compound of formula (I) into a further compound of formula (I);
(ii) preparing a salt of the compound of formula (I) and/or a solvate thereof.

10. A pharmaceutical composition comprising a compound of formula (I), as defined in claim 1, or a pharmaceutically acceptable salt thereof and/or a pharmaceutically acceptable solvate thereof, and a pharmaceutically acceptable carrier.

11. A compound of formula (I), as defined in claim 1, or a pharmaceutically acceptable salt thereof and/or a pharmaceutically acceptable solvate thereof, for use as an active therapeutic substance.

12. A compound of formula (I), as defined in claim 1, or a pharmaceutically acceptable salt thereof and/or a pharmaceutically acceptable solvate thereof, for use in the treatment of and/or prophylaxis of arrhythmia and ischeamic rhythm disorders.

13. The use of a compound of formula (I) as defined in claim 1, or a pharmaceutically acceptable salt thereof and/or a pharmaceutically acceptable solvate thereof, for the manufacture of a medicament for the treatment of arrhythmia and ischeamic rhythm disorders.

## Patentansprüche

1. Verbindung der Formel (I) 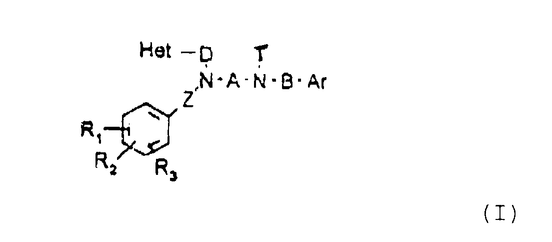 oder ein Salz davon, oder ein Solvat davon, in der
A und B unabhängig voneinander jeweils eine C₁-C₄ n-Alkylengruppe bedeuten, bei der jedes Kohlenstoffatom gegebenenfalls mit einer C₁-C₆ Alkylgruppe substituiert ist;
D CO, SO₂, NH-CO oder CH₂CO bedeutet;
T ein Wasserstoffatom, eine Alkyl-, Alken- oder Cycloalkylgruppe bedeutet; und
Het substituierte oder unsubstituierte Furanyl-, Pyranyl-, Thienyl-, Thiazolyl-, Imidazolyl- oder Triazolylreste bedeutet, oder die entsprechenden benzolkondensierten Furanyl-, Pyranyl-, Thienyl-, Thiazolyl-, Imidazolyl- oder Triazolyl-, Indoyl, Oxoindoyl, Indenyl-, Isoindenyl-, Indazolyl-, Indolizinyl- oder Benzofuranzanylreste;
mögliche Substituenten für die Het-Gruppe 1 bis 5 Substituenten umfassen, ausgewählt aus einer Nitrogruppe, einem Halogenatom, einem Alkylsulfonamido-, Alkylamido-, 1H-Imidazolyl-, Alkyl- und Haloalkylrest;
R₁, R₂ und R₃ unabhängig voneinander jeweils ein Wasserstoffatom, eine Alkylgruppe, eine OH-Gruppe oder eine Alkoxygruppe bedeuten, oder, falls sie an benachbarte Kohlenstoffatome gebunden sind, zwei der Reste R₁, R₂ und R₃ zusammen mit den Kohlenstoffatomen, an die sie gebunden sind, einen kondensierten heterozyklischen Ring mit fünf oder sechs Atomen bilden können, wobei eines, zwei oder drei der Atome (ein) Sauerstoffatom(e) oder (ein) Stickstoffatom(e) ist/sind;
Z eine Bindung, eine CH₂, (CH₂)₂ oder X-CH₂-CH₂-Gruppe bedeutet, in der X ein Sauerstoff- oder Schwefelatom ist; und
Ar eine substituierte oder unsubstituierte Arylgruppe bedeutet, wobei die möglichen Substituenten die oben definierten Reste R₁, R₂ und R₃ sind.

2. Verbindung nach Anspruch 1, in der A (CH₂)₃, B CH₂CH₂ und T CH₃ bedeuten.

3. Verbindung nach Anspruch 1 oder 2, in der D CO, SO₂, NH-CO oder CH₂CO bedeutet.

4. Verbindung nach einem der Ansprüche 1 bis 3, in der D CO bedeutet.

5. Verbindung nach einem der Ansprüche 1 bis 4, in der einer oder zwei der Reste R₁, R₂ und R₃ eine Alkoxygruppe bedeutet/bedeuten und der/die verbleibende(n) Rest(e) jeweils unabhängig voneinander ein Wasserstoffatom bedeutet/bedeuten.

6. Verbindung nach einem der Ansprüche 1 bis 5, in der Het eine Furanyl- oder Thienylverbindung bedeutet.

7. Verbindung nach einem der Ansprüche 1 bis 6, in der Het 4-Nitro-2-thiophenyl, 5-Nitro-2-furanyl oder 5-Benzofurazanyl bedeutet.

8. Verbindung nach Anspruch 1, nämlich
N-(3,4-dimethoxyphenyl)-N-[3-[[2-(3,4-dimethoxyphenyl)-ethyl]methylamino]propyl]-4-nitro-2-thiophencarboxamid;
N-(3,4-dimethoxyphenyl)-N-[3-[[2-(3,4-dimethoxyphenyl)ethyl]methylamino]propyl]-5-nitro-2-furancarboxamid; und
N-(3,4-dimethoxyphenyl)-N-[3-[[2-(3,4-dimethoxyphenyl)ethyl]methylamino]propyl]-5-benzofurazancarboxamid Hydrochloridhydrat; oder ein Salz davon, oder ein Solvat davon.

9. Verfahren zur Herstellung einer Verbindung der Formel (I), umfassend die Reaktion einer Verbindung der Formel (II) 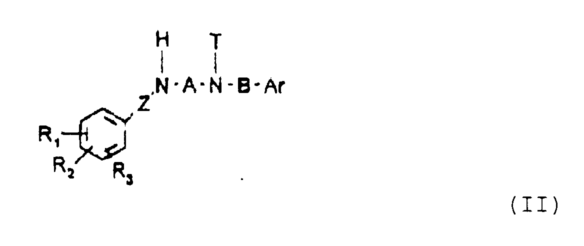 in der A, B, T, Z, R₁, R₂, R₃ und Ar wie vorstehend mit Bezug auf Formel (I) definiert sind, mit einer Verbindung der Formel (III)
Het-X (III)
in der Het wie vorstehend mit Bezug auf Formel (I) definiert ist und
(a) für Verbindungen der Formel (I), in denen D CO oder CO₂ ist, X CO.L₁ bzw. SO₂.L₁ bedeutet, wobei L₁ eine Abgangsgruppe oder -atom wie z.B. ein Halogenatom darstellt; oder
(b) für Verbindungen der Formel (I), in denen D NH-CO ist, X N=C=O bedeutet; und anschließend, wenn nötig, Durchführen eines oder mehrerer der folgenden möglichen Schritte:
(i) Überführen einer Verbindung der Formel (I) in eine weitere Verbindung der Formel (I);
(ii) Herstellen eines Salzes der Verbindung der Formel (I) und/oder eines Solvats davon.

10. Arzneimittel umfassend eine Verbindung der Formel (I) wie in Anspruch 1 definiert oder ein pharmazeutisch verträgliches Salz davon und/oder ein pharmazeutisch verträgliches Solvat davon, sowie einen pharmazeutisch verträglichen Träger.

11. Verbindung der Formel (I) wie in Anspruch 1 definiert oder ein pharmazeutisch verträgliches Salz davon und/oder ein pharmazeutisch verträgliches Solvat davon zur Verwendung als therapeutischer Wirkstoff.

12. Verbindung der Formel (I) wie in Anspruch 1 definiert oder ein pharmazeutisch verträgliches Salz davon und/oder ein pharmazeutisch verträgliches Solvat davon zur Verwendung in der Behandlung und/oder Prophylaxe von Arrhytmie und ischämischen Rhythmusstörungen.

13. Verwendung einer Verbindung der Formel (I) wie in Anspruch 1 definiert oder eines pharmazeutisch verträglichen Salzes davon und/oder eines pharmazeutisch verträglichen Solvats davon zur Herstellung eines Medikaments zur Behandlung von Arrhytmie und ischämischen Rhythmusstörungen.

## Revendications

1. Composé de formule (I) : 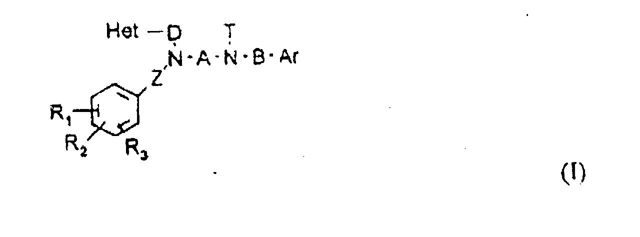 ou un de ses sels, ou bien un de ses produits de solvatation, formule dans laquelle
A et B représentent chacun indépendamment un groupe n-alkylène en C₁ à C₄ dans lequel chaque atome de carbone est facultativement substitué avec un groupe alkyle en C₁ à C₆ ;
D représente un groupe CO, SO₂, NH-CO ou CH₂CO ;
T représente l'hydrogène, un groupe alkyle, alcène ou cycloalkyle ; et
Het représente un groupe substitué ou non substitué : furannyle, pyrannyle, thiényle, thiazolyle, imidazolyle, triazolyle ou les équivalents benzo-condensés des groupes furannyle, pyrannyle, thiényle, thiazolyle, imidazolyle ou triazolyle, indolyle, oxo-indolyle, indényle, iso-indényle, indazolyle, indolizinyle ou benzofurazanyle ;
les substituants facultatifs pour le groupement Het comprennent 1 à 5 substituants choisis dans la liste des substituants nitro, halogéno, alkylsulfonamido, alkylamido, 1H-imidazolyle, alkyle et halogénalkyle ;
R₁, R₂ et R₃ représentent chacun indépendamment H, un groupe alkyle, OH ou alkoxy ou, s'ils sont fixés à des atomes de carbone adjacents, deux quelconques de R₁, R₂ et R₃, conjointement avec les atomes de carbone auxquels ils sont fixés, peuvent former un noyau hétérocyclique condensé de cinq ou six atomes dans lequel un, deux ou trois desdits atomes consistent en atomes d'oxygène ou d'azote ;
Z représente une liaison, un groupe CH₂, (CH₂)₂ ou X-CH₂-CH₂ dans lequel X représente O ou S ; et
Ar représente un groupe aryle substitué ou non substitué, dans lequel les substituants facultatifs sont les substituants R₁, R₂ et R₃ définis ci-dessus.

2. Composé suivant la revendication 1, dans lequel A représente un groupe (CH₂)₃, B représente un groupe CH₂CH₂ et T représente un groupe CH₃.

3. Composé suivant la revendication 1 ou la revendication 2, dans lequel D représente un groupe CO, SO₂ ou NH-CO ou bien CH₂CO.

4. Composé suivant l'une quelconque des revendications 1 à 3, dans lequel D représente un groupe CO.

5. Composé suivant l'une quelconque des revendications 1 à 4, dans lequel un ou deux des groupes R₁, R₂ et R₃ représentent un groupe alkoxy et le ou les symboles restants représentent chacun indépendamment H.

6. Composé suivant l'une quelconque des revendications 1 à 5, dans lequel Het représente un groupe furannyle ou thiényle.

7. Composé suivant l'une quelconque des revendications 1 à 6, dans lequel Het représente un groupe 4-nitro-2-thiophényle, 5-nitro-2-furannyle ou 5-benzofurazanyle.

8. Composé suivant la revendication 1, qui consiste en :
N-(3,4-diméthoxyphényl)-N-[3-[[2-(3,4-diméthoxyphényl]éthyl]méthylamino]propyl]-4-nitro-2-thiophène-carboxamide ;
N-((3,4-diméthoxyphényl)-N-[3-[[2-(3,4-diméthoxyphényl]éthyl]méthylamino]propyl]-5-nitro-2-furanne-carboxamide ;
hydrate de chlorhydrate de N-((3,4-diméthoxyphényl)-N-[3-[[2-(3,4-diméthoxyphényl]éthyl]méthylamino]propyl]-5-benzofurazanecarboxamide ; ou un des ses sels, ou bien un de ses produits de solvatation.

9. Procédé pour la préparation d'un composé de formule (I), procédé qui comprend la réaction d'un composé de formule (II) : 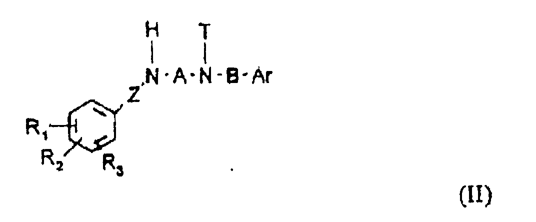 dans laquelle A, B, T, Z, R₁, R₂, R₃ et Ar répondent aux définitions mentionnées en rapport avec la formule (I), avec un composé de formule (III) :
Het-X (III)
dans laquelle Het répond à la définition mentionnée en rapport avec la formule (I) ; et :
(a) pour des composés de formule (I) dans laquelle D représente un groupe CO ou SO₂, X représente un groupe CO.L₁ ou SO₂-L₁, respectivement, dans lequel L₁ représente un groupe ou atome partant tel qu'un atome d'halogène ; ou
(b) pour des composés de formule (I) dans laquelle D représente un groupe NH-CO, X représente un groupe N=C=O ; et ensuite, si besoin, la mise en oeuvre d'une ou plusieurs des étapes facultatives suivantes :
(i) la transformation d'un composé de formule (I) en un autre composé de formule (I) ;
(ii) la préparation d'un sel du composé de formule (I) et/ou d'un de ses produits de solvatation.

10. Composition pharmaceutique comprenant un composé de formule (I), répondant à la définition suivant la revendication 1, ou un de ses sels pharmaceutiquement acceptables et/ou un de ses produits de solvatation pharmaceutiquement acceptables, et un support pharmaceutiquement acceptable.

11. Composé de formule (I), répondant à la définition suivant la revendication 1, ou un de ses sels pharmaceutiquement acceptables et/ou un de ses produits de solvatation pharmaceutiquement acceptables, destinés à être utilisé comme substance thérapeutique active.

12. Composé de formule (I), répondant à la définition suivant la revendication 1, ou un des ses sels pharmaceutiquement acceptables et/ou un de ses produits de solvatation pharmaceutiquement acceptables, destiné à être utilisé dans le traitement et/ou la prophylaxie de l'arythmie et de troubles ischémiques du rythme.

13. Utilisation d'un composé de formule (I) répondant à la définition suivant la revendication 1, ou d'un de ses sels pharmaceutiquement acceptables et/ou d'un de ses produits de solvatation pharmceutiquement acceptables, pour la production d'un médicament destiné au traitement de l'arythmie et de troubles ischémiques du rythme.
